(19) Europäisches Patentamt
European Patent Office
Office européen des brevets

(11) Veröffentlichungsnummer : **0 344 112 B1**

(12) **EUROPÄISCHE PATENTSCHRIFT**

(45) Veröffentlichungstag der Patentschrift :
**20.01.93 Patentblatt 93/03**

(21) Anmeldenummer : **89810366.8**

(22) Anmeldetag : **18.05.89**

(51) Int. Cl.[5] : **C07C 69/017,** C07C 69/18, C07C 317/22, C07C 323/21, C07F 7/08, C07D 495/06, // (C07D495/06, 339:00, 339:00)

# (54) Substituierte Bisacyloxynaphtacene und Verfahren zur Herstellung von Tetrathiotetracenen.

(30) Priorität : **27.05.88 CH 2008/88**
**19.07.88 CH 2754/88**

(43) Veröffentlichungstag der Anmeldung :
**29.11.89 Patentblatt 89/48**

(45) Bekanntmachung des Hinweises auf die Patenterteilung :
**20.01.93 Patentblatt 93/03**

(84) Benannte Vertragsstaaten :
**CH DE FR GB IT LI NL**

(56) Entgegenhaltungen :
**JOURNAL OF CHEMICAL RESEARCH, 1981, Nr. 12, Seite 368, London (GB); LIM KEOW BEE et al: "Diels-alder reactions of7,7-dichloro-3,4-dimethylenebicyclo-[4.1.0]heptane with quinones. Synthesis of tetracenequinones"**

(56) Entgegenhaltungen :
**CHEMICAL AND PHARMACEUTICAL BULLETIN, Band 26, Nr. 12, Dezember 1978, Seiten 3820-3824, Tokyo (JP); T. KAMETANI et al.:"Studies on the syntheses of tetracycline derivatives. III. Synthetic approach to adriamycin - synthesis of the linear tetracyclicskeleton having the same BC ring system with adriamycin"**

(73) Patentinhaber : **CIBA-GEIGY AG**
**Klybeckstrasse 141**
**CH-4002 Basel (CH)**

(72) Erfinder : **Baumann, Marcus, Dr.**
**Arlesheimerstrasse 18**
**CH-4053 Basel (CH)**
Erfinder : **Mayer, Carl, W., Dr.**
**Steingrubenweg 224**
**CH-4125 Riehen (CH)**
Erfinder : **Wernet, Wolfgang, Dr.**
**Schillerstrasse 40**
**W-7800 Freiburg (DE)**
Erfinder : **Fischer, Walter, Dr.**
**Vogesenstrasse 77**
**CH-4153 Reinach (CH)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft in 1-, 2-, 3-, 4-, 7-, 8-, 9- und/oder 10-Stellung substituierte 5,12-Acyloxynaphthacene, ein Verfahren zu deren Herstellung und ein Verfahren zur Herstellung von unsubstituierten oder entsprechend substituierten 5,6,11,12-Tetrathiotetracenen.

Bei der Herstellung von Tetrathio- und Tetraselenotetracenen sind Naphthacen-5,12-dione wichtige Zwischenprodukte. Die Reduktion der Dione führt je nach Reaktionsbedingungen und Reaktionszeit zu Dihydrotetracenen, Tetracenen oder Gemischen hiervon. Die Tetracene können zu 5,11-bzw. 6,12-Dichlortetracenen chloriert werden.

Die direkte Umsetzung von Dihydrotetracen mit Schwefel ergibt in mässigen Ausbeuten Tetrathiotetracen [Ch. Marschalk, Bull. Soc. Chim. France S. 931 und 1122 (1939)]. Auch die direkte Umsetzung von Tetracenen und Dichlortetracenen mit Schwefel ergibt Tetrathiotetracene [EP-A-0 153 905 und Ch. Marschalk, Bull. Soc. Chim. France, S. 427 (1948)]. Die Herstellung möglichst reiner Tetrathiotetracene nach diesen Verfahren setzt unter anderem voraus, dass man von reinen Tetracenen ausgeht. Die Reduktion von Naphthacendionen ergibt jedoch oft Gemische mit Hydroxy- und Dihydroxytetracenen, wobei zudem die Ausbeuten nicht befriedigend sind. Zudem reagieren Hydroxy- und Dihydroxytetracene mit Schwefel nicht zu Tetrathiotetracenen.

5,12-Diacetoxytetracen ist von T. Kametani in Chem. Pharm. Bull. 26(12), S. 3820-3824 (1978) beschrieben worden.

L. Bee et al. in J. Chem. Res./Synop. 1981 vol, 12, S.368 beschreiben 5,12-Acetyloxy-7-Methyltetracen als Zwischenprodukt in einem mehrstufigen Verfahren.

Es wurde nun gefunden, dass man 5,12-Diacyloxy-tetracene direkt mit Schwefel zu 5,6,11,12-Tetrathiotetracenen umsetzen kann. Die 5,12-Diacyloxytetracene sind hierbei durch einfache reduktive Acylierung in hohen Ausbeuten und Reinheiten zugänglich.

Ein Gegenstand der Erfindung sind Verbindungen der Formel I

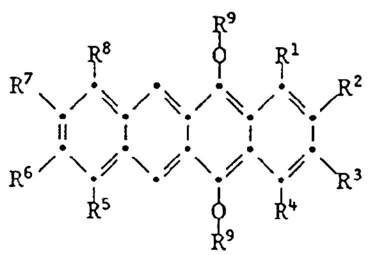

(I),

worin $R^1$ bis $R^8$ unabhängig voneinander H und mindestens eines von $R^1$ bis $R^8$ ein Substituent aus der Gruppe -F, $-Si(C_1\text{-}C_4\text{-Alkyl})_3$, $-COOR^{10}$; oder je zwei benachbarte Reste von $R^1$ bis $R^8$ -CO-O-CO-; oder mindestens eines von $R^1$ bis $R^8$ unsubstituiertes oder mit -F, -OH-, -CN, $C_1$-$C_{12}$-Alkoxy, $C_1$-$C_{12}$-Acyloxy oder $-COOR^{10}$ substitiertes $C_1$-$C_{20}$-Alkyl $-(X)_p-$, $C_2$-$C_{20}$-Alkenyl$-(X)_p-$, $C_2$-$C_{20}$-Alkinyl$-(X)_p-$, $C_3$-$C_8$-Cycloalkyl $-(X)_p-$, $C_1$-$C_{12}$-Alkyl-$C_3$-$C_8$-cycloalkyl$-(X)_p-$, $C_3$-$C_8$-Cycloalkyl-$CH_2$$-(X)_p-$, $C_1$-$C_{12}$-Alkyl-$C_3$-$C_8$-cycloalkyl-$CH_2$$-(X)_p-$, $C_6$-$C_{10}$-Aryl-X-, $C_7$-$C_{20}$-Alkaryl-X-, $C_7$-$C_{12}$-Aralkyl$-(X)_p-$ oder $C_8$-$C_{20}$-Alkaralkyl$-(X)_p-$ oder

$$-Y-(C_mH_{2m}-O)_n-R^{10}$$

darstellen;
$R^{10}$ für H oder $C_1$-$C_{18}$-Alkyl steht, X -O-, -S-, -SO- oder $-SO_2-$ und Y -O- oder -S- bedeuten, und p für 0 oder 1, m für eine Zahl von 2 bis 6 und n für eine Zahl von 2 bis 20 stehen;
und $R^9$ unsubstituiertes oder mit -F substituiertes $C_1$-$C_4$-Acyl darstellt, mit Ausnahme von 5,12-Acetyloxy-7-Methyltetracen.

Bedeuten $R^1$ bis $R^8$ $-Si(C_1\text{-}C_4\text{-Alkyl})_3$, so kann es sich bei der Alkylgruppe um Methyl, Ethyl, n- oder i-Propyl oder n- und i-Butyl handeln. Besonders bevorzugt ist $-Si(CH_3)_3$.

Bedeuten $R^1$ bis $R^8$ $-COOR^{10}$, so kann $R^{10}$ H oder lineares oder verzweigtes Alkyl mit bevorzugt 1 bis 12, besonders 1 bis 8 C-Atomen sein. Beispiele für Alkyl sind Methyl, Ethyl, und die Isomeren von Propyl, Butyl,

Pentyl, Hexyl, Heptyl, Octyl, Nonyl, Decyl, Undecyl, Dodecyl, Tetradecyl, Hexadecyl und Octadecyl.

$R^1$ bis $R^8$ können in Rahmen der vorangehenden Definitionen substituiert sein, bevorzugt ein-bis dreifach, besonders ein-oder zweifach. Bei den Substituenten kann es sich z.B. um lineares oder verzweigtes Alkoxy mit vorzugsweise 1 bis 6, besonders 1 bis 4 C-Atomen handeln. Besonders bevorzugt sind Ethoxy und Methoxy.

Bei den Substituenten kann es sich z.B. auch um Acyloxy mit vorzugsweise 1 bis 6 C-Atomen handeln, z.B. $C_1$-$C_6$-Alkyl-CO-O- oder Benzyloxy. Beispiele sind Formyloxy, Acetoxy, Trifluoracetoxy, Propionyloxy, Acryloxy, Methacryloxy und Butanoyloxy.

Bei den Substituenten kann es sich ferner z.B. um $-COOR^{10}$ handeln, worin $R^{10}$ bevorzugt für H oder $C_1$-$C_4$-Alkyl steht.

In einer bevorzugten Ausführungsform können $R^1$ bis $R^8$ mit -OH, -F, $C_1$-$C_4$-Alkoxy, -COO($C_1$-$C_4$-Alkyl) und $C_1$-$C_6$-Akyl-CO-O- substituiert sein.

$R^1$ bis $R^8$ können $C_1$-$C_{20}$-Alkyl$-(X)_p-$ sein. Die Alkylgruppe kann linear oder verzweigt sein und enthält bevorzugt 1 bis 18, besonders 1 bis 12 und insbesondere 1 bis 8 C-Atome. Beispiel für Alkylgruppen sind zuvor erwähnt worden.

$R^1$ bis $R^8$ können $C_2$-$C_{20}$-Alkenyl$-(X)_p-$ sein, worin p bevorzugt 1 ist, X bevorzugt für -O- steht und die Alkenylgruppe bevorzugt eine terminale Alkengruppe enthält. Das Alkenyl kann linear oder verzweigt sein und bevorzugt 2 bis 18, besonders 2 bis 12 und insbesondere 2 bis 6 C-Atome enthalten. Beispiele sind Ethenyl, Allyl, Prop-1-en-1- oder -2-yl, But-1-en-1- oder -2- oder -3- oder -4-yl, But-2-en-1- oder -2-yl, Pent-1-en- oder Pent-2-en-1- oder -2- oder -3- oder -4- oder -5-yl, Hex-1-en- oder Hex-2-en- oder Hex-3-en-1- oder -2- oder -3- oder -4- oder -5- oder -6-yl, Heptenyl, Octenyl, Nonenyl, Decenyl, Undecenyl, Dodecenyl, Tetracenyl, Hexadecenyl und Octadecenyl.

$R^1$ bis $R^8$ können $C_2$-$C_{20}$-Alkinyl$-(X)_p-$ sein, worin p bevorzugt 1 ist, X bevorzugt für -O- steht und die Alkinylgruppe bevorzugt eine terminale Alkingruppe enthält. Das Alkinyl kann linear oder verzweigt sein und bevorzugt 2 bis 18, besonders 2 bis 12 und insbesondere 2 bis 6 C-Atome enthalten. Beispiele sind Ethinyl, Propargyl, But-1-in-3- oder -4-yl, Pent-1-in-3- oder -4- oder -5-yl, Hex-1-in-3- oder -4- oder -5- oder -6-yl, Hex-2-in-1- oder -4- oder -5- oder -6-yl, Hex-3-in-1- oder -2-yl, Heptinyl, Octinyl, Noninyl, Decinyl, Undecinyl und Dodecinyl.

$R^1$ bis $R^8$ können $C_3$-$C_8$-Cycloalkyl$-(X)_p-$ sein, worin p bevorzugt für 1 steht und das Cycloalkyl bevorzugt 3 bis 6, besonders 5 oder 6 C-Atome enthält. Beispiele sind Cyclopropyl, Cyclobutyl, Cyclopentyl, Cyclohexyl, Cycloheptyl und Cyclooctyl.

$R^1$ bis $R^8$ können ($C_1$-$C_{12}$-Alkyl)-$C_3$-$C_8$-cycloalkyl$-(X)_p-$ sein, worin das Alkyl bevorzugt 1 bis 6 C-Atome enthält, p bevorzugt für 1 steht und das Cycloalkyl bevorzugt Cyclopentyl oder Cyclohexyl ist. Beispiele sind Methyl- oder Ethylcyclopentyl oder -cyclohexyl, Methylcyclopropyl und Methylcyclobutyl.

$R^1$ bis $R^8$ können $C_3$-$C_8$-, bevorzugt $C_5$- oder $C_6$-Cycloalkyl-$CH_2$$-(X)_p-$ sein, worin p bevorzugt 1 ist.

$R^1$ bis $R^8$ können ($C_1$-$C_{12}$-Alkyl)-$C_3$-$C_8$-cycloalkyl-$CH_2$$-(X)_p-$ sein, worin p bevorzugt 1 ist, die Alkylgruppe bevorzugt 1 bis 6 C-Atome enthält und das Cycloalkyl bevorzugt Cyclopentyl oder Cyclohexyl ist. Beispiele sind Methylcyclohexylmethyl oder -ethyl.

Bei $R^1$ bis $R^8$ in der Bedeutung von $C_6$-$C_{10}$-Aryl-X- stellt das Aryl besonders Naphthyl und insbesondere Phenyl dar. Bei $R^1$ bis $R^8$ in der Bedeutung von $C_7$-$C_{20}$-Alkaryl-X- handelt es sich vorzugsweise um ($C_1$-$C_{14}$-Alkyl)-phenyl-X-, wobei die Alkylgruppe bevorzugt 1 bis 8, besonders 1 bis 4 C-Atome enthält.

Bei $R^1$ bis $R^8$ in der Bedeutung von $C_7$-$C_{12}$-Aralkyl$-(X)_p-$ handelt es sich bevorzugt um Benzyl-X- oder Phenylethyl-X-.

Bei $R^1$ bis $R^8$ in der Bedeutung von $C_8$-$C_{20}$-Alkaralkyl$-(X)_p-$ handelt es sich bevorzugt um ($C_1$-$C_{13}$-Alkyl)benzyl-X-, wobei die Alkylgruppe insbesondere 1 bis 6 C-Atome enthält. Beispiele sind Methylbenzyl-X- und Ethylbenzyl-X-.

Bei $R^1$ bis $R^8$ in der Bedeutung von

$$-Y-(C_mH_{2m}-O)_n-R^{10}$$

stellt Y bevorzugt -O- dar, m ist bevorzugt 2 oder 3 und n bevorzugt eine Zahl von 2 bis 12, besonders 2 bis 6, und $R^{10}$ steht bevorzugt für H oder $C_1$-$C_4$-Alkyl. Bei der $C_mH_{2m}$-Gruppe handelt es sich besonders um Ethylen oder 1,2-Propylen.

$R^9$ ist bevorzugt unsubstituiertes oder mit F substituiertes $C_1$- oder $C_2$-Acyl. Beispiele sind Butanoyl, Propionyl, Mono- oder Difluoracetoxy, Formyloxy und besonders Acetoxy und Trifluoracetoxy.

Eine bevorzugte Ausführungsform sind solche Verbindungen der Formel I, worin $R^1$ bis $R^8$ unabhängig voneinander H und mindestens eines von $R^1$ bis $R^8$ ein Substituent aus der Gruppe -F, $-Si(CH_3)_3$, $-COO(C_1$-$C_{12}$-Alkyl), unsubstituiertes oder mit -F, $C_1$-$C_6$-Alkoxy, $C_1$-$C_6$-Acyloxy oder -COO-($C_1$-$C_{12}$-Alkyl) substituiertes

$C_1$-$C_{18}$-Alkyl $-(X)_p-$ , $C_2$-$C_{18}$-Alkenyl$-(X)_p-$ , $C_2$-$C_{12}$-Alkinyl$-(X)_p-$ , $C_6$-$C_{10}$-Aryl-X-, $C_7$-$C_{18}$-Alkaryl-X-, $C_7$-$C_{10}$-Aralkyl$-(X)_p-$ , $C_8$-$C_{18}$-Alkaralkyl$-(X)_p-$ oder

$$-Y-(CH_2CH_2O)_n-R^{10},$$

darstellen, $R^{10}$ für H oder $C_1$-$C_6$-Alkyl steht, X -O-, -S-, -SO- oder $-SO_2$- und Y -O- oder -S- bedeuten, m für 2 oder 3 und n für eine Zahl von 2 bis 12 stehen, und p 0 oder 1 bedeutet, und $R^9$ unsubstituiertes oder mit -F substituiertes Acetyl darstellt.

Eine andere bevorzugte Ausführungsform sind solche Verbindungen der Formel I, worin $R^1$ und $R^4$ bis $R^8$ H darstellen; $R^2$ -F, $-CF_3$, $-COO(C_1$-$C_{18}$-Alkyl), unsubstituiertes oder mit -OH, $-COO(C_1$-$C_6$-Alkyl) oder $C_1$-$C_6$-Acyloxy substituiertes $C_1$-$C_{18}$-Alkyl-X;

$$-Y-(CH_2CH_2O)_n-R^{10},$$

worin Y -O- oder -S-, $R^{10}$ H oder $C_1$-$C_6$-Alkyl und n eine Zahl von 2 bis 12 bedeuten; $C_2$-$C_{12}$-Alkenyl-$CH_2$-X-; $C_2$-$C_{12}$-Alkinyl-$CH_2$-X-; Phenyl-X-; $C_1$-$C_{12}$-Alkylphenyl-X-; Benzyl-X-; $C_1$-$C_{12}$-Alkylbenzyl-X bedeutet und $R^3$ H ist oder unabhängig die Bedeutung von $R^2$ hat, und X -O-, -S-, -SO- oder $-SO_2$-ist.

$R^1$ bedeutet als Substituent besonders $-CF_3$ oder $-COO(C_1$-$C_{12}$-Alkyl).

Bevorzugte Ausführungsformen sind auch solche Verbindungen der Formel I, worin a) $R^4$, $R^5$ und $R^8$ für H stehen, oder b) $R^1$, $R^4$, $R^5$ und $R^8$ für H stehen, oder c) $R^2$ und/oder $R^3$ einen Substituenten und $R^1$ und $R^4$ bis $R^8$ H bedeuten, oder d) $R^6$ und/oder $R^7$ einen Substituenten und $R^1$ bis $R^5$ und $R^8$ H bedeuten.

Ein weiterer Gegenstand der Erfindung ist ein Verfahren zur Herstellung von Verbindungen der Formel I, das dadurch gekennzeichnet ist, dass man in einem Lösungsmittel ein substituiertes Naphthacen-5,12-dion der Formel II

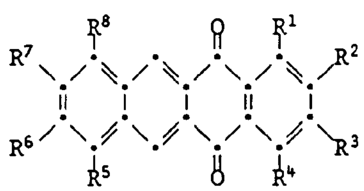

(II),

worin $R^1$ bis $R^8$ die zuvor angegebenen Bedeutungen haben, unter reduzierenden Bedingungen mit einem Anhydrid der Formel $R^9CO$-O-$OCR^9$, worin $R^9$ die zuvor angegebene Bedeutung hat, umsetzt.

Die reduktive Acylierung unter Verwendung von reduzierenden Metallen, z.B. Zink, ist bekannt und von T. Kametani in Chem. Pharm. Bull 26(12), S. 3820-3825 (1978) beschrieben worden. Die Reduktion kann auch elektrochemisch oder katalytisch mit Edelmetallkatalysatoren wie z.B. Pt oder Pd vorgenommen werden.

Die katalytische Reduktion oder die Reduktion mit Metallen wird zweckmässig in Gegenwart eines Alkalimetallsalzes der dem Anhydrid der Formel $R^9CO$-O-$OCR^9$ zugrunde liegenden Carbonsäure $R^9COOH$ durchgeführt. Das Alkalimetall kann z.B. Li, Na, K, Rb oder Cs sein.

Die Reaktion wird in Gegenwart eines inerten Lösungsmittels durchgeführt, z.B. Carbonsäureestern, wie z.B. Essigsäuremethyl- oder -ethylester. Hierdurch verläuft die Reaktions unter milderen Bedingungen und mit höherer Ausbeute als bei den bekannten Verfahren.

Die Reaktionstemperatur kann z.B. 20 bis 100°C betragen. Vorteilhaft wird eine Temperatur im Bereich der Raumtemperatur, z.B. 20 bis 40°C gewählt.

Die substituierten Naphthacen-5,12-dione der Formel II sind teilweise bekannt oder sie können mit den nachfolgend beschriebenen Verfahren hergestellt werden.

Die Verbindungen der Formel II können z.B. hergestellt werden, indem man nach Friedel-Craft ein Naphthalindicarbonsäureanhydrid der Formel IV

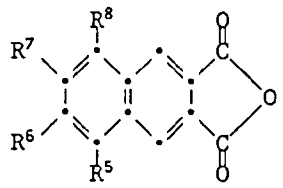

(IV)

in Gegenwart einer Lewissäure mit einem Benzol der Formel V

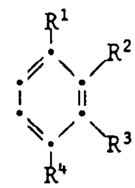

(V)

umsetzt, wobei $R^1$ bis $R^8$ die zuvor angegebenen Bedeutungen haben, und Verbindungen der Formel I, worin mindestens eines von $R^1$ bis $R^8$ -F bedeutet, nukleophil substituiert. Für die nukleophile Substitution geeignete Verbindungen sind besonders solche der Formel ($R^1$ bis $R^8$)-X-H, worin X -O-, -S-, -SO- oder $-SO_2-$ sind, Malonsäureester oder -nitrile und Phenylessigsäurenitril. Diese nukleophilen Verbindungen können in Form ihrer Alkalisalze, z.B. Li-, Na- oder K-Salze verwendet werden. Ebenso ist es möglich, die nukleophile Substitution in Gegenwart von Basen, wie z.B. Alkalilaugen oder -carbonaten durchzuführen.

Die Verbindungen der Formel II können auch hergestellt werden, indem man in einer Diels-Alder-Reaktion eine Verbindung der Formel VI

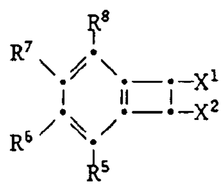

(VI)

worin $R^5$, $R^6$, $R^7$ und $R^8$ die zuvor angegebenen Bedeutungen haben und $X^1$ und $X^2$ unabhängig voneinander für -Cl, -Br oder -J stehen, unter nachfolgender Abspaltung von $HX^1$ und $HX^2$ mit einer Verbindung der Formel VII

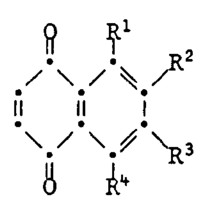

(VII),

worin $R^1$, $R^2$, $R^3$ und $R^4$ die zuvor angegebenen Bedeutungen haben, umsetzt. In den Substituenten $R^1$ bis $R^8$ ist p bevorzugt 1.

Die Reaktion wird vorteilhaft bei Temperaturen von 50 bis 250°C, bevorzugt 80 bis 200°C durchgeführt. Zweckmässig wird ein inertes Lösungsmittel verwendet, zum Beispiel polare aprotische Lösungsmittel. Einige Beispiele sind aromatische Kohlenwasserstoffe (Benzol, Toluol, Xylol, Chlorbenzol und Dichlorbenzol), Nitrile (Acetonitril), Ether (Dibutylether, Dioxan, Tetrahydrofuran, Ethylenglykol- oder Diethylenglykoldimethylether). Die Isolierung und Reinigung kann nach üblichen Methoden erfolgen, z.B. Kristallisation, Sublimation oder Chromatographie.

Verbindungen der Formel VI sind teilweise bekannt (siehe z.B. H.P. Cava et al., J. Am. Chem. Soc., S. 1701 (1957) und J.W. Barton et al., J. Chem. Soc. Perkin Trans. 1, S. 967-971 (1986)), bzw. nach analogen Verfahren herstellbar. Die für die Herstellung benötigten substituierten 1,2-Bis(dichlor- oder dibrommethyl)benzole sind ebenfalls teilweise bekannt oder durch übliche elektrophile oder nukleophile Substitutionsreaktionen entsprechender o-Dimethylbenzole, und deren anschliessenden Chlorierung oder Bromierung mit z.B. N-Chlor- oder N-Bromsuccinimid erhältlich.

Die p-Naphthochinone der Formel VII sind bekannt und z.B. durch nukleophile Substitution von gegebenenfalls geschützten und substituierten Halogen- oder Nitro-1,4-naphthochinonen mit z.B. den zuvor beschriebenen Verbindungen in Gegenwart von Alkalimetallverbindungen ($K_2CO_3$, $Cs_2CO_3$, KOH, NaOH, $NaNH_2$, $NaOCH_3$, $NaOC_2H_5$) oder mit Alkalimetall-Verbindungen z.B. von Li, K, Na, Rb oder Cs, erhältlich. Halogen- und Nitro-naphthochinone sind z.B. in Houben-Weyl, Chinone I, Band 7/3b (1977) beschreiben. Die Naphthochinone der Formel VII können auch in bekannter Weise durch elektrophile oder nukleophile Substitution von gegebenenfalls substituierten Naphthalinen, Dihydro- oder Tetrahydronaphthalinen und anschliessende Umwandlung in die substituierten 1,4-Naphthochinone hergestellt werden.

Die Verbindungen der Formel II können auch hergestellt werden, indem man 1,2-Bis(dihalogenmethyl)benzole der Formel

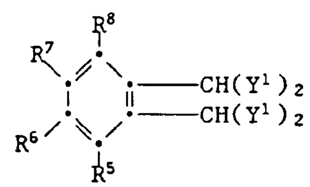 ,

worin $Y^1$ für Cl, Br oder I steht, in Gegenwart von NaI in einem organischen Lösungsmittel mit einer Verbindung de Formel VII umsetzt. Diese Methode ist von J.W. McOmie in Synthesis, S. 416-417 (1973) beschrieben.

Verbindungen der Formel II können auch erhalten werden, indem man Anthracen-1,4-chinone der Formel

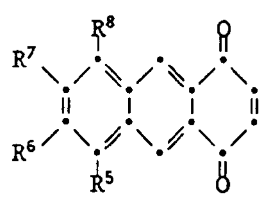

mit einem α-Pyron der Formel VIII

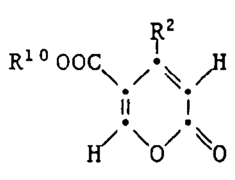 (VIII),

oder einem Butadien der Formel IX

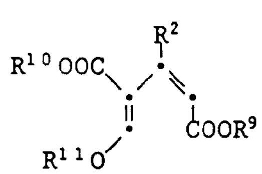 (IX)

umsetzt, wobei $R^{11}$ $C_1$-$C_6$-Alkyl ist, $R^{10}$ die zuvor angegebene Bedeutung hat und bevorzugt $C_1$-$C_6$-Alkyl ist. Diese Methode und die Herstellung von α-Pyronen ist in der US-PS 4,617,151 und der EP-A-0 195 743 beschrieben.

Verbindungen der Formeln VIII und IX sind z.B. folgendermassen erhältlich, wobei $X^1$ ein Alkalimetall bedeutet:

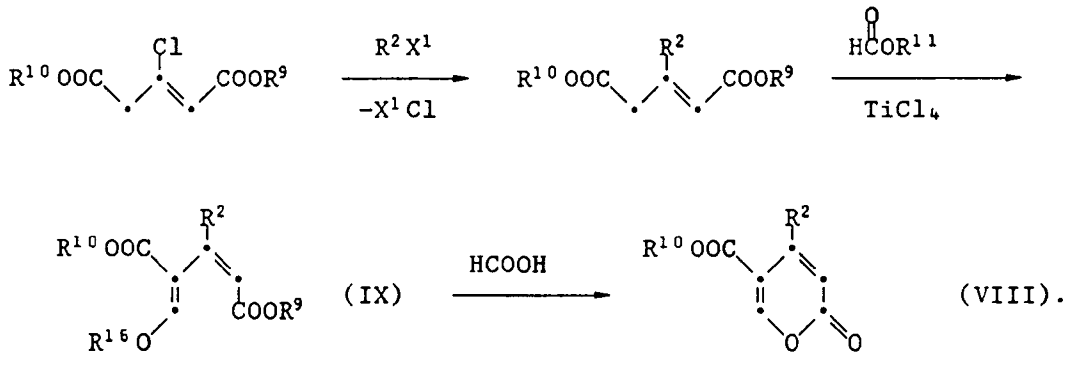


Wenn $R^1$ bis $R^8$ einen Polyoxaalkylenrest bedeuten, so erhält man solche Verbindungen auch durch Umsetzung von Verbindungen der Formel I mit $R^1$ bis $R^8$ gleich Hydroxyalkyloxy mit Epoxiden. Ferner ist es möglich die Reste $R^1$ bis $R^8$ durch klassische Reaktionen abzuwandeln, wie z.B. Hydrolyse, Ver- oder Umesterung, Amidierung, Oxidation oder Reduktion. Carbonsäureester können in bekannter Weise mit $HF/SF_4$ in die Trifluormethylderivate umgewandelt werden.

Die Verbindungen der Formel I werden in kurzen Reaktionszeiten in hohen Ausbeuten und in hoher Reinheit erhalten. Sie eignen sich überraschend zur direkten Umsetzung mit Schwefel unter Bildung von Tetrathiotetracenen.

Ein weiterer Gegenstand ist ein Verfahren zur Herstellung von 5,6,11,12-Tetrathiotetracenen der Formel III

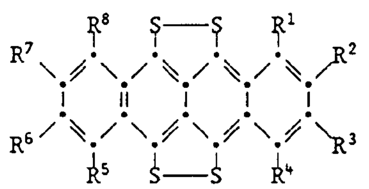
(III),

worin $R^1$ bis $R^8$ unabhängig voneinander H oder einen Substituenten aus der Gruppe -F, $-Si(C_1\text{-}C_4\text{-Alkyl})_3$, $-COOR^{10}$; oder je zwei benachbarte Rest von $R^1$ bis $R^8$ -CO-O-CO-; oder $R^1$ bis $R^8$ einen Substituenten aus der Gruppe unsubstituiertes oder mit -F, -OH-, $C_1$-$C_{12}$-Alkoxy, $C_1$-$C_{12}$-Acyloxy, oder $-COOR^{10}$ substitiertes $C_1$-$C_{20}$-Alkyl$-(X)_p-$, $C_3$-$C_8$-Cycloalkyl$-(X)_p-$, $C_1$-$C_{12}$-Alkyl-$C_3$-$C_8$-cycloalkyl$-(X)_p-$, $C_3$-$C_8$-Cycloalkyl-$CH_2-(X)_p-$, $C_1$-$C_{12}$-Alkyl-$C_3$-$C_8$-cycloalkyl-$CH_2-(X)_p-$, $C_6$-$C_{10}$-Aryl-X-, $C_7$-$C_{20}$-Alkaryl-X-, $C_7$-$C_{12}$-Aralkyl$-(X)_p-$ oder $C_8$-$C_{20}$-Alkaralkyl$-(X)_p-$ oder

$$-Y-(C_mH_{2m}-O)_n-R^{10}$$

darstellen;

$R^{10}$ für H oder $C_1$-$C_{18}$-Alkyl steht, X -O-, -S-, -SO- oder $-SO_2-$ und Y -O- oder -S- bedeuten, und p für 0 oder 1, m für eine Zahl von 2 bis 6 und n für eine Zahl von 2 bis 20 stehen; dadurch gekennzeichnet, dass man eine Verbindung der Formel Ia

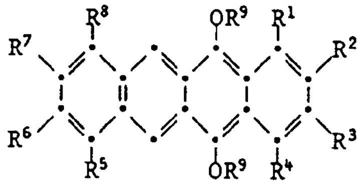

(Ia),

worin $R^1$ bis $R^8$ die zuvor angegebenen Bedeutungen haben und $R^9$ unsubstituiertes oder mit -F substituiertes $C_1$-$C_4$-Acyl darstellt, in Gegenwart katalytischer Mengen einer Sulfonsäure mit Schwefel umsetzt.

Die Reaktion wird zweckmässig in einem hochsiedenden Lösungsmittel durchgeführt. Geeignete Lösungsmittel sind besonders halogenierte aromatische Kohlenwasserstoffe, z.B. Chlorbenzol, Dichlorbenzole und Trichlorbenzole, aber auch Nitrobenzol oder Dowtherm A.

Die Reaktion wird ferner zweckmässig unter Inertgasatmosphäre durchgeführt, z.B. unter Verwendung von Edelgasen (Helium, Neon, Argon) oder Stickstoff.

Die Reaktionstemperatur beträgt vorteilhaft 100 bis 250°C, besonders 150 bis 250°C. Es ist zweckmässig, bei Rückflusstemperatur des gewählten Lösungsmittels zu arbeiten.

Die Menge an Sulfonsäure kann 0,001 bis 10 Mol-%, bevorzugt 0,001 bis 5 Mol-% und besonders 0,01 bis 2 Mol-% betragen, bezogen auf die Menge der Verbindungen der Formel Ia.

Geeignete Sulfonsäuren sind z.B. organische Sulfonsäuren, besonders aromatische Sulfonsäuren. Beispiele sind Methan-, Ethan-, Propan-, Butan-, Hexan-, Trifluormethan- oder Benzolsulfonsäure und besonders p-Toluolsulfonsäure.

Mit dem erfindungsgemässen Verfahren erhält man die Tetrathiotetracene der Formel III in guten Ausbeuten. Sie können nach der Isolierung mit Hilfe üblicher Methoden gereinigt werden, z.B. durch Umkristallisation oder Sublimation. Die Isolierung erfolgt im allgemeinen durch Entfernen des Lösungsmittels, Auswaschen des Rückstandes mit einem Nichtlösungsmittel und Trocknen.

Aus den Verbindungen der Formel III können mit Elektronenacceptoren elektrisch leitende Charge-Transferkomplexe (CT-Komplexe) hergestellt werden. Mit deren funktionellen Substituenten kann man sie an Polymere binden, z.B. als Seitengruppen in Polymere einbauen (vgl. US-PS 4,617,151). Die CT-Komplexe eignen sich auch zur Herstellung z.B. von antistatischen Beschichtungen photographischer Filmelemente, Magnetbänder, elektrophotographischer Filmelemente und elektronischer Komponenten (siehe US-PS 3,634,336). Die chalkogenierten Tetracene weisen ferner elektrochrome Eigenschaften auf; sie können für elektrochrome Displays verwendet werden. Sie eignen sich auch als laser-optische Datenspeicher [Nach. Chem. Techn. Lab. 35, S. 255 ff (1987)] und als Anodenmaterial in organischen Solid-State-Batterien (EP-A-0 090 598). CT-Komplexe von substituierten Tetrathio- oder Tetraselenotetracenen können zur Erzielung antistatischer Eigenschaften auch in thermoplastische, duroplastische oder elastomere Polymere eingearbeitet werden. Hierzu werden zweckmässig z.B. die substituierten Tetrathio- bzw. Tetraselenotetracene zusammen mit einem löslichen Polymer oder einer Vorstufe davon und einem Elektronenacceptor, z.B. einem Halogen bildenden Mittel (organische halogenierte Verbindungen wie z.B. Bromoform, Trichlorbrommethan, Tetrabrommethan, Hexachlorpropan, Perchlorbutadien, 1,3- oder 1,4-Dichlor-2-buten, 1,4-Bis(trichloromethyl)-benzol, Iodacetonitril, Iodoform, Tetrachlorethylen, Perchlorcyclobutadien, N-Chlor-, -brom oder -iodsuccinimid) gegebenenfalls zusammen mit einem weiteren inerten Lösungsmittel gelöst und das überschüssige Halogen bildende Mittel und das Lösungsmittel bei höherer Temperatur verdampft. Die gebildete Zusammensetzung enthält im Polymer ein Netzwerk nadelförmiger Kristalle des CT-Komplexes, wenn das chalkogenierte Tetracen unsubstituiert ist oder kleine Substituenten (z.B. F, $CH_3$, $CF_3$) enthält. Solche Zusammensetzungen weisen eine hohe elektrische Leitfähigkeit auf. Diese kann noch verbessert werden, wenn man ein substituiertes Tetrathio- oder Tetraselenotetracen der Formel III mitverwendet, das kein solches Netzwerk bildet und das in der Polymermatrix in feiner Verteilung vorliegt, da solche substituierten Tetrathio-oder Tetraselenotetracene keine oder nur eine geringe Kristallisationstendenz im Polymer besitzen.

Die nachfolgenden Beispiele erläutern die Erfindung näher.

A) Herstellungsbeispiele

Beispiele 1-22:

10,35 mMol 2-substituiertes Naphthacen-5,12-dion, 40 ml Essigsäureethylester, 25 ml Acetanhydrid und 31,05 mMol Kaliumacetat werden unter Rühren mit 31,05 mMol Zinkpulver versetzt. Nach 40 Minuten Rühren bei

25°C wird das Reaktionsgemisch filtriert und der Rückstand viermal mit $CH_2Cl_2$ gewaschen. Die Filtrate werden eingedampft und der Rückstand aus $CH_2Cl_2$/Pentan und dann aus Toluol umkristallisiert. Ausbeuten und Schmelzpunkte der erhaltenen 2-substitierten 5,12-Diacetoxynaphthacene sind in Tabelle 1 angegeben.

Tabelle 1

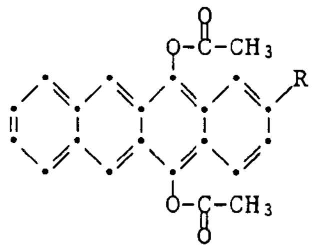

| Beispiel Nr. | R | Ausbeute (%) | Schmelzpunkt (°C) |
|---|---|---|---|
| 1 | $-OCH_3$ | 81 | 247-252 |
| 2 | $-OCH_2CH_3$ | 79 | 150-153 |
| 3 | $-OCH_2-CH(C_2H_5)(CH_2)_3CH_3$ | 71 | 130-135 |
| 4 | $-O-n-C_8H_{17}$ | 84 | 107-111 |
| 5 | $-OCH(CH_3)_2$ | 61 | 215-218 |
| 6 | $-O-n-C_{18}H_{37}$ | 49 | 155-157 |
| 7 | $-OCH_2CH=CH_2$ | 74 | 200-201 |
| 8 | $-OCH_2C\equiv CH$ | 59 | 213-215 |
| 9 | $-OCH_2CH_2OH$ | 47 | 189-193 |
| 10 | $-O(CH_2)_4OH$ | 36 | 181-183 |
| 11 | $-O(CH_2)_2O(CH_2)_2-OH$ | 28 | 149-152 |
| 12 | $-SO_2CH_3$ | 69 | >250 |
| 13 | $-SCH_2CH_3$ | 77 | 130-135 |
| 14 | -S- 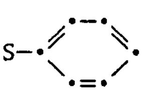 | 79 | 145-150 |
| 15 | $-SO_2-$ 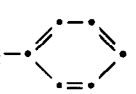 | 82 | >220 (Zersetzung) |
| 16 | F | 73 | 170-175 |
| 17 | $-C(CH_3)(COOC_2H_5)_2$ | 63 | 230 (Zersetzung) |
| 18 | $-CF_3$ | 91 | >250 |
| 19 | $-COOCH_3$ | 81 | 185-190 |
| 20 | $-COO(CH_2)_3CH_3$ | 73 | 195-196 |
| 21 | $-COO(CH_2)_7CH_3$ | 61 | 143-145 |
| 22 | $-COOCH_2CH(C_2H_5)(CH_2)_3CH_3$ | 45 | 149-150 |

Die entsprechenden 2-substituierten Naphthacen-5,12-dione der Beispiele 1-15 und 17 sind durch nukleophile Substitution von 2-Fluornaphtacen-5,12-dion erhältlich.

2-(Trifluormethyl-)-naphthacen-5,12-dion:

5,65 g (25 mMol) 6-(Trifluormethyl-)-1,4-naphthochinon, 9,82 g (ca. 37 mMol) 1,2-Dibrombenzocyclobuten (verunreinigt mit etwas 2-Brom-1-jodbenzocyclobuten) und 100 ml Xylol werden während 16 Stunden mit einem Wasserabscheider am Rückfluss gehalten. Das Gemisch wird abgekühlt und der Niederschlag abfiltriert umd mit Xylol gewaschen. Ausbeute 5,82 g (71 %); Smp. 253-254°C.

Analog wird bei der Herstellung der in den nachfolgenden Beispielen 24-26 verwendeten 2,3-Bis-(trifluormethyl-)-naphthacen-5,12-dion (Ausbeute 59 %; Smp. >280°C); 1-(Trifluormethyl-)-naphthacen-5,12-dion-3-carbonsäuremethylester (Ausbeute 60 %; Smp. 234-235°C) und 2-Ethoxy-naphthacen-5,12-dion-3-carbonsäuremethylester (Ausbeute 30 %; Smp. 192-194°C) verfahren.

Beispiele 23-27:

1,65 mMol 2-substituiertes Naphthacen-5,12-dion, 5 ml Essigsäureethylester, 4,96 mMol Kaliumacetat und 3 ml Acetanhydrid werden unter Zugabe von 0,1 g Pd/C (5 %) bei 20-25°C während 35 Minuten hydriert. Das Gemisch wird filtriert und der Rückstand dreimal mit $CH_2Cl_2$ ausgewaschen. Die Filtrate werden eingedampft und der Rückstand aus $CH_2Cl_2$/Pentan umkristallisiert. Ausbeuten und Schmelzpunkte der erhaltenen 5,12-Diacetoxynaphthacene sind in Tabelle 2 angegeben.

Tabelle 2

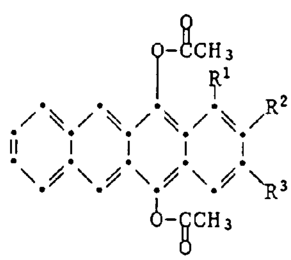

| Beispiel Nr. | $R^1$ | $R^2$ | $R^3$ | Ausbeute % | Schmelzpunkt (°C) |
|---|---|---|---|---|---|
| 23 | H | $-OCH_2CH_3$ | H | 70 | 149-153 |
| 24 | H | $-CF_3$ | $-CF_3$ | 76 | >250 |
| 25 | $-CF_3$ | H | $-COOCH_3$ | 18 | >280 |
| 26 | H | $-OCH_2CH_3$ | $-COOC_2H_5$ | 65 | 208-210 |
| 27[a] | H | $-CH_3$ | $-CH_3$ | 40 | >250 |

[a] 2,3-Dimethyl-naphtacen-5,12-dion wird erhalten durch Kondensation von 1,2-Dibrombenzocyclobuten mit 6,7-Dimethylnaphthalin-1,4-dion unter auschliessender in situ-Abspaltung von 2HBr.

Beispiel 28:

In eine durch eine D3 Glasfritte getrennte Zelle, ausgestattet mit einer als Rührer ausgebildeten Edelstahlkathode und einer Pt-Anode, wird eine Lösung von 400 ml Acetanhydrid und 11,1 g (0,04 mol) Tetrabutylammoniumchlorid gegeben. Unter Stickstoff werden dann 3 g (0,67 mMol) 2-(4-Hydroxybutoxy)-naphthacen-5,12-dion zugegeben und bei einer Stromstärke von 20 mA wird 20 Stunden elektrolysiert. Danach wird die Lösung

des Kathodenraumes in Eiswasser eingetragen und mit gesättigter Natriumcarbonatlösung neutralisiert. Das ausgefallene Produkt wird abfiltriert und mit Wasser gewaschen. Ausbeute 3,8 g (90,5 %) 2-(4'-Acetoxybutoxy)-5,12-diacetoxy-naphthacen. Massenspektrum: $M^+$ = 474

| | | | | |
|---|---|---|---|---|
| Elementaranalyse: | berechnet: | %C 70,87 | %H 5,52 | %O 23,60 |
| | gefunden: | %C 70,20 | %H 5,60 | %O 23,66 |

Beispiel 29:

2-Ethoxy-5,12-bis-(trifluoracetoxy)-naphthacen:

2 g (6,62 mMol) 2-Ethoxynaphthacen-5,12-dion, 55 ml Trifluoressigsäureanhydrid, 2,7 g Trifluoressigsäure-Natriumsalz, 20 ml Essigsäureethylester und 1,08 g (16,54 mMol) Zinkstaub werden während 3 Std. bei 25-30°C gerührt. Das Gemisch wird abfiltriert und der Rückstand mit Tetrahydrofuran/$CH_2Cl_2$ mehrmals nachgewaschen. Die Filtrate werden eingedampft und der Rückstand mit Diethylether verrührt. Die Kristalle werden abfiltriert und aus Toluol umkristallisiert: Ausbeute 2,83 g (86 %); Smp. 165-70°C.

Beispiele 30-36:

In einem 250 ml Kolben mit Rückflusskühler und Gaseinleitungsrohr werden unter leichtem Argonfluss 1,83 mMol substituiertes Naphthacen-5,12-diacetat, 15,8 mVal $S_8$ und 0,026 mMol p-Toluolsulfonsäure in 100 ml 1,2,4-Trichlorbenzol 5 1/2 Stunden am Rückfluss erhitzt. Danach wird die dunkelgrüne Lösung am Hochvakuum eingedampft.

Das Rohprodukt wird über eine Kieselgelflashsäule (240 g Kieselgel, ∅ 7 cm) mit $CCl_4$ chromatographiert. [Das Kieselgel muss zuvor mit $CCl_4$, das 2 % Triethylamin enthält, behandelt und darauf mit reinem $CCl_4$ gewaschen werden, bis das Eluat wieder neutral ist.] Die dunkelgrün gefärbten Fraktionen enthalten das gereinigte 2-substituierte 5,6,11,12-Tetrathiotetracen. Spektrale Daten und Ausbeuten sind in Tabelle 3 angegeben.

Tabelle 3

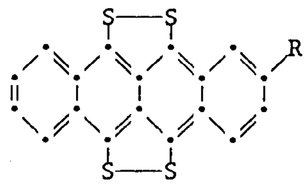

| Beispiel Nr. | R | Massenspektrum ($M^+$) | $\lambda_{max}$ (nm) in 1,2,4-Trichlorbenzol | Ausbeute (%) |
|---|---|---|---|---|
| 30 | $-OC_2H_5$ | 396 | 700 | 24[a)] |
| 31 | $-O-n-C_8H_{17}$ | 480 | 699 | 50[b)] |
| 32 | $-OCH_2CH(C_2H_5)(CH_2)_3CH_3$ | 480 | 698 | 8[a)] |
| 33 | $-O-n-C_{18}H_{37}$ | 620 | 698 | 50[a)] |
| 34 | $-C(CH_3)(COOC_2H_5)_2$ | 524 | 704 | 10[a)] |
| 35 | $-COO(CH_2)_3CH_3$ | 452 | 745 | 82 % (roh) |
| 36 | $-SC_2H_5$ | 412 | 711 | 3 %[a)] |

a) sublimiert

b) chromatographiert

Beispiel 37:

In einem 100 ml Kölbchen mit Rückflusskühler und Gaseinlassrohr werden unter leichtem Argonfluss 251 mg (0,61 mMol) 2-Trifluormethylnaphthacen-5,12-diacetat, 78 mg (2,43 mVal) $S_8$ und 2 mg (0,01 mMol) p-Toluolsulfonsäure in 35 ml 1,2,4-Trichlorbenzol 20 Stunden am Rückfluss erhitzt. Nach dem Abkühlen wird am Hochvakuum (HV) das Lösungsmittel abgedampft, der Rückstand mit Hexan ausgekocht, das schwarze Pulver abfiltriert und am HV bei 60°C getrocknet. Man erhält 203 mg (79 %) Rohprodukt.

Dieses wird bei 190°C (1,3 x $10^{-4}$ mbar) sublimiert, wobei 67,5 mg (35,6 %) reines 2-Trifluormethyl-5,6,11,12-tetrathiotetracen erhalten werden (schwarze Nädelchen) . Massenspektrum: $M^+$ = 420.
$\lambda_{max}$ (1,2,4-Trichlorbenzol): 725, 665, 484 nm.

Beispiel 38:

2,3-Trifluormethyl-5,6,11,12-tetrathiotetracen

Es wird wie in Beispiel 37 verfahren und 2,3-Trifluormethylnaphtacen-5,12-diacetat verwendet. Ausbeute: 75,6 mg (30 %) nach Sublimation.
Massenspektrum: $M^+$ = 488.
$\lambda_{max}$ (1,2,4-Trichlorbenzol): 755 nm.

B) Anwendungsbeispiele:

Beispiel 39:

Elektrochromie

In eine Elektrochromiezelle, bestehend aus einer Teflonmembran und in je 0,5 mm Abstand einer Anode und Kathode aus ITO-Glas, werden zur Anodenseite 1 mg 2,3-Di(trifluormethyl)-5,6,11,12-tetrathioetracen und 100 mg $LiClO_4$, gelöst in 5 ml Aceton, und zur Kathodenseite eine Lösung von 1 mg 2,3-Di(trifluormethyl)-5,6,11,12-tetrathiotetracen-perchlorat (CT-Komplex, Herstellung siehe US-PS 3,634,336) und 100 mg $LiClO_4$ in 5 ml Ace-

ton eingefüllt. Nach Anlegen einer Spannung von 2 Volt wechseln innerhalb weniger Sekunden die Farben von grün nach rotviolett auf der Anodenseite und von rotviolett nach grün auf der Kathodenseite. Durch Umpolung der Spannung werden in beiden Zellenhälften die ursprünglichen Farben erhalten. Der gleiche Effekt wird beobachtet, wenn man Nitrobenzol oder Dimethylformamid als Lösungsmittel verwendet.

## Patentansprüche

1. Verbindungen der Formel I

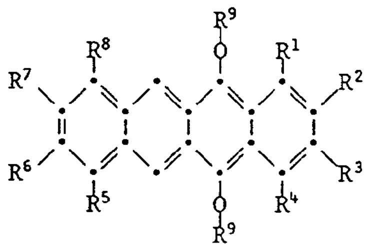

(I),

worin $R^1$ bis $R^8$ unabhängig voneinander H und mindestens eines von $R^1$ bis $R^8$ ein Substituent aus der Gruppe -F, $-Si(C_1\text{-}C_4\text{-Alkyl})_3$, $-COOR^{10}$; oder je zwei benachbarte Reste von $R^1$ bis $R^8$ -CO-O-CO-; oder mindestens eines von $R^1$ bis $R^8$ unsubstituiertes oder mit -F, -OH-, -CN, $C_1$-$C_{12}$-Alkoxy, $C_1$-$C_{12}$-Acyloxy oder $-COOR^{10}$substitiertes $C_1$-$C_{20}$-Alkyl $-(X)_p-$, $C_2$-$C_{20}$-Alkenyl$-(X)_p-$, $C_2$-$C_{20}$-Alkinyl$-(X)_p-$, $C_3$-$C_8$-Cycloalkyl$-(X)_p-$, $C_1$-$C_{12}$-Alkyl-$C_3$-$C_8$-cycloalkyl$-(X)_p-$, $C_3$-$C_8$-Cycloalkyl-$CH_2$$-(X)_p-$, $C_1$-$C_{12}$-Alkyl-$C_3$-$C_8$-cycloalkyl-$CH_2$$-(X)_p-$, $C_6$-$C_{10}$-Aryl-X-, $C_7$-$C_{20}$-Alkaryl-X-, $C_7$-$C_{12}$-Aralkyl$-(X)_p-$ oder $C_8$-$C_{20}$-Alkaralkyl$-(X)_p-$ oder

$$-Y-(C_mH_{2m}-O)_n-R^{10}$$

darstellen;
$R^{10}$ für H oder $C_1$-$C_{18}$-Alkyl steht, X -O-, -S-, -SO- oder $-SO_2-$ und Y -O- oder -S- bedeuten, und p für 0 oder 1, m für eine Zahl von 2 bis 6 und n für eine Zahl von 2 bis 20 stehen;
und $R^9$ unsubstituiertes oder mit -F substituiertes $C_1$-$C_4$-Acyl darstellt, mit Ausnahme von 5,12-Acetyloxy-7-Methyltetracen.

2. Verbindungen gemäss Anspruch 1, worin $R^1$ bis $R^8$ unabhängig voneinander H und mindestens eines von $R^1$ bis $R^8$ ein Substituent aus der Gruppe -F, $-Si(CH_3)_3$, $-COO(C_1\text{-}C_{12}\text{-Alkyl})$, unsubstituiertes oder mit -F, $C_1$-$C_6$-Alkoxy, $C_1$-$C_6$-Acyloxy oder $-COO(C_1\text{-}C_{12}\text{-Alkyl})$ substituiertes $C_1$-$C_{18}$-Alkyl$-(X)_p-$ $C_2$-$C_{18}$-Alkenyl$-(X)_p-$ $C_2$-$C_{12}$-Alkinyl$-(X)_p-$, $C_6$-$C_{10}$-Aryl-X-, $C_7$-$C_{18}$-Alkaryl-X-, $C_7$-$C_{10}$-Aralkyl $-(X)_p-$ $C_8$-$C_{18}$-Alkaralkyl$-(X)_p-$ oder

$$-Y-(C_mH_{2m}-O)_n-R^{10}$$

darstellen, $R^{10}$ für H oder $C_1$-$C_6$-Alkyl steht, X -O-, -S-, -SO- oder $-SO_2-$ und Y -O- oder -S-bedeuten, m für 2 oder 3 und n für eine Zahl von 2 bis 12 stehen, und p 0 oder 1 bedeutet, und $R^9$ unsubstituiertes oder mit -F substituiertes Acetyl darstellt.

3. Verbindungen gemäss Anspruch 1, worin $R^4$, $R^5$ und $R^8$ für H stehen.

4. Verbindungen gemäss Anspruch 1, worin $R^1$, $R^4$, $R^5$ und $R^8$ für H stehen.

5. Verbindungen gemäss Anspruch 1, worin $R^2$ und/oder $R^3$ einen Substituenten und $R^1$ und $R^4$ bis $R^8$ H bedeuten.

6. Verbindungen gemäss Anspruch 1, worin $R^6$ und/oder $R^7$ einen Substituenten und $R^1$ bis $R^5$ und $R^8$ H bedeuten.

7. Verbindungen gemäss Anspruch 1, worin $R^1$ als Substituent $-CF_3$ oder $COO(C_1$-$C_{12}$-Alkyl) ist.

8. Verbindungen gemäss Anspruch 1, worin $R^1$ und $R^4$ bis $R^8$ H darstellen, $R^2$ -F, $-CF_3$, $-COO(C_1$-$C_{18}$-Alkyl), unsubstituiertes oder mit -OH, $-COO(C_1$-$C_6$-Alkyl) oder $C_1$-$C_6$-Acyloxy substituiertes $C_1$-$C_{18}$-Alkyl-X;

$$-Y\!-\!(CH_2CH_2O)_n\!-\!R^{10} ,$$

worin Y -O- oder -S-, $R^{10}$ H oder $C_1$-$C_6$-Alkyl und n eine Zahl von 2 bis 12 bedeuten; $C_2$-$C_{12}$-Alkenyl-$CH_2$-X-; $C_2$-$C_{12}$-Alkinyl-$CH_2$-X-; Phenyl-X-; $C_1$-$C_{12}$-Alkylphenyl-X-; Benzyl-X-; $C_1$-$C_{12}$-Alkylbenzyl-X bedeutet und $R^3$ H ist oder unabhängig die Bedeutung von $R^2$ hat, und X -O-, -S-, -SO- oder $-SO_2$-ist.

9. Verfahren zur Herstellung von Verbindungen der Formel I gemäss Anspruch 1, dadurch gekennzeichnet, dass man in einem Lösungsmittel ein substituiertes Naphthacen-5,12-dion der Formel II

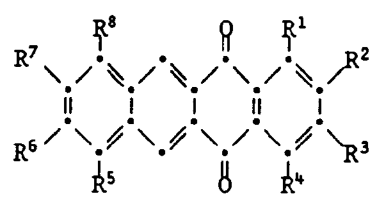

(II),

worin $R^1$ bs $R^8$ die zuvor angegebenen Bedeutungen haben, unter reduzierenden Bedingungen mit einem Anhydrid der Formel $R^9CO$-O-$OCR^9$, worin $R^9$ die in Anspruch 1 angegebene Bedeutung hat, umsetzt.

10. Verfahren gemäss Anspruch 9, dadurch gekennzeichnet, dass das Lösungsmittel ein Carbonsäureester ist.

11. Verfahren gemäss Anspruch 9, dadurch gekennzeichnet, dass die reduzierenden Bedingungen elektrochemisch, katalytisch oder durch Zusatz von reduzierenden Metallen erzeugt werden.

12. Verfahren zur Herstellung von 5,6,11,12-Tetrahiotetracenen der Formel III

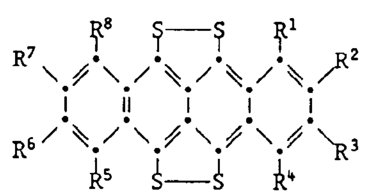

(III),

worin $R^1$ bis $R^8$ unabhängig voneinander H oder einen Substituenten aus der Gruppe -F, $-Si(C_1$-$C_4$-Alkyl$)_3$, $-COOR^{10}$ oder je zwei benachbarte Rest von $R^1$ bis $R^8$ -CO-O-CO-; oder $R^1$ bis $R^8$ einen Substituenten aus der Gruppe unsubstituiertes oder mit -F, -OH-, $C_1$-$C_{12}$-Alkoxy, $C_1$-$C_{12}$-Acyloxy oder $-COOR^{10}$ substitiertes $C_1$-$C_{20}$-Alkyl$-(X)_p-$ ,$C_3$-$C_8$-Cycloalkyl$-(X)_p-$ , $C_1$-$C_{12}$-Alkyl-$C_3$-$C_8$-cycloalkyl$-(X)_p-$ ,$C_3$-$C_8$-Cycloalkyl-$CH_2$$-(X)_p-$ ,$C_1$-$C_{12}$-Alkyl-$C_3$-$C_8$-cycloalkyl-$CH_2$$-(X)_p-$ , $C_6$-$C_{10}$-Aryl-X-, $C_7$-$C_{20}$-Alkaryl-X-, $C_7$-$C_{12}$-Aralkyl$-(X)_p-$ oder $C_8$-$C_{20}$-Alkaralkyl$-(X)_p-$ oder

$$-Y\!-\!(C_mH_{2m}\!-\!O)_n\!-\!R^{10}$$

darstellen;
$R^{10}$ für H oder $C_1$-$C_{18}$-Alkyl steht, X -O-, -S-, -SO- oder -$SO_2$- und Y -O- oder -S- bedeuten, und p für 0 oder 1, m für eine Zahl von 2 bis 6 und n für eine Zahl von 2 bis 20 stehen; dadurch gekennzeichnet, dass man eine Verbindung der Formel Ia

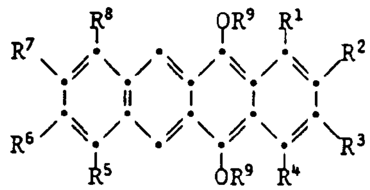

(Ia),

worin $R^1$ bis $R^8$ die zuvor angegebenen Bedeutungen haben und $R^9$ unsubstituiertes oder mit -F substituiertes $C_1$-$C_4$-Acyl darstellt, in Gegenwart katalytischer Mengen einer Sulfonsäure mit Schwefel umsetzt.

**13.** Verfahren gemäss Anspruch 12, dadurch gekennzeichet, dass man die Reaktion in Gegenwart eines hochsiedenden Lösungsmittels durchführt.

**14.** Verfahren gemäss Anspruch 12, dadurch gekennzeichnet, dass man die Reaktion unter Inertgasatmosphäre durchführt.

**15.** Verfahren gemäss Anspruch 12, dadurch gekennzeichnet, dass es sich bei der Sulfonsäure um eine aromatische Sulfonsäure handelt.

**16.** Verfahren gemäss Anspruch 15, dadurch gekennzeichnet, dass es sich um p-Toluolsulfonsäure handelt.

**17.** Verfahren gemäss Anspruch 12, dadurch gekennzeichnet, dass die Menge der Sulfonsäure 0,001 bis 10 Mol-% beträgt, bezogen auf die Menge der Verbindung der Formel Ia.

## Claims

**1.** A compound of the formula I

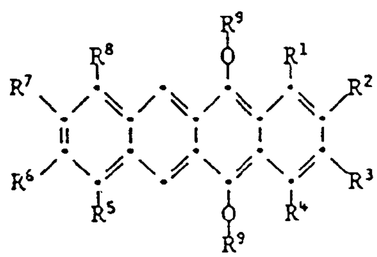

(I),

in which $R^1$ to $R^8$ independently of one another are H and at least one of $R^1$ to $R^8$ is a substituent belonging to the group -F, -Si($C_1$-$C_4$alkyl)$_3$ or -$COOR^{10}$; or each pair of adjacent radicals of $R^1$ to $R^8$ is -CO-O-CO-; or at least one of $R^1$ to $R^8$ is $C_1$-$C_{20}$alkyl-$(X)_p$-, $C_2$-$C_{20}$alkenyl-$(X)_p$-, -$C_2$-$C_{20}$-alkynyl-$(X)_p$-, $C_3$-$C_8$cycloalkyl-$(X)_p$-, ($C_1$-$C_{12}$alkyl-$C_3$-$C_8$cycloalkyl-$(X)_p$-, $C_3$-$C_8$-cycloalkyl-$CH_2$-$(X)_p$-, $C_1$-$C_{12}$alkyl-$C_3$-$C_8$cycloalkyl-$CH_2$-$(X)_p$-, $C_6$-$C_{10}$aryl-X-, $C_7$-$C_{20}$alkaryl-X-, $C_7$-$C_{12}$aralkyl-$(X)_p$- or $C_8$-$C_{20}$alkaralkyl-$(X)_p$- or -Y-$(C_mH_{2m}$-$O)_n$-$R^{10}$ each of which is unsubstituted or substituted by - F, -OH, -CN, $C_1$-$C_{12}$alkoxy, $C_1$-$C_{12}$acyloxy or -$COOR^{10}$; $R^{10}$ is H or $C_1$-$C_{18}$alkyl, X is -O-, -S-, -SO- or -$SO_2$- and Y is -O- or -S-, and p is 0 or 1, m is a number from 2 to 6 and n is a number from 2 to 20; and $R^9$ is $C_1$-$C_4$acyl which is unsubstitued or substituted by -F, with the exception of 5,12-acetyloxy-7-methyltetracene.

**2.** A compound according to claim 1, in which $R^1$ to $R^8$ independently of one another are H and at least one of $R^1$ to $R^8$ is a substituent belonging to the group -F, -Si$(CH_3)_3$, -COO-($C_1$-$C_{12}$-alkyl), $C_1$-$C_{18}$alkyl-$(X)_p$-, $C_2$-$C_{18}$alkenyl-$(X)_p$-, $C_2$-$C_{12}$alkynyl-$(X)_p$-, $C_6$-$C_{10}$aryl-X-, $C_7$-$C_{18}$alkaryl-X-, $C_7$-$C_{10}$aralkyl-$(X)_p$-, $C_8$-$C_{18}$alkaralkyl-$(X)_p$- or -Y-$(C_mH_{2m}$-O-$)_n$-$R^{10}$ each of which is unsubstituted or subtituted by -F, $C_1$-$C_6$alkoxy, $C_1$-

$C_6$acyloxy or -COO-($C_1$-$C_{12}$-alkyl), $R^{10}$ is H or $C_1$-$C_6$alkyl, X is -O-, -S-, -SO- or -$SO_2$- and Y is -O- or -S-, m is 2 or 3 and n is a number from 2 to 12 and p is 0 or 1 and $R^9$ is acetyl which is unsubstituted or substituted by -F.

3. A compound according to claim 1, in which $R^4$ $R^5$ and $R^8$ are H.

4. A compound according to claim 1, in which $R^1$, $R^4$, $R^5$ and $R^8$ are H.

5. A compound according to claim 1, In which $R^2$ and/or $R^3$ are a substituent and $R^1$ and $R^4$ to $R^8$ are H.

6. A compound according to claim 1, in which $R^6$ and/or $R^7$ are a substituent and $R^1$ to $R^5$ and $R^8$ are H.

7. A compound according to claim 1, in which $R^1$, as a substituent, is -$CF_3$ or -COO-($C_1$-$C_{12}$-alkyl).

8. A compound according to claim 1, in which $R^1$ and $R^4$ to $R^8$ are H; $R^2$ is -F, - $CF_3$, -COO-($C_1$-$C_{18}$alkyl) or $C_1$-$C_{18}$alkyl-X- which is unsubstituted or substituted by -OH, -COO-($C_1$-$C_6$alkyl) or $C_1$-$C_6$acyloxy; -Y-$(CH_2CH_2O)_n$-$R^{10}$ in which Y is -O- or -S-, $R^{10}$ is H or $C_1$-$C_6$alkyl and n is a number from 2 to 12; $C_2$-$C_{12}$alkenyl-$CH_2$-X-; $C_2$-$C_{12}$alkynyl-$CH_2$-X-; phenyl-X-; $C_1$-$C_{12}$alkylphenyl-X-; benzyl-X-; or $C_1$-$C_{12}$alkylbenzyl-X- and $R_3$ is H or independently is the same as $R_2$, and X is -O-, -S-, -SO- or -$SO_2$-.

9. A process for the preparation of compounds of the formula I according to claim 1, which comprises reacting a substituted naphthacene-5,12-dione of the formula II

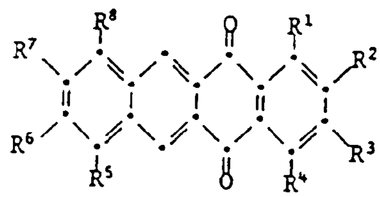
(II)

in which $R^1$ to $R^8$ are as defined above, in a solvent and under reducing conditions, with an anhydride of the formula $R^9$CO-O-OC$R^9$ in which $R^9$ is as defined above.

10. A process according to claim 9, wherein the solvent is a carboxylic acid ester.

11. A process according to claim 9, wherein the reducing conditions are produced electrochemically, catalytically or by the addition of reducing metals.

12. A process for the preparation of 5,6,11,12-tetrathiotetracenes of the formula III

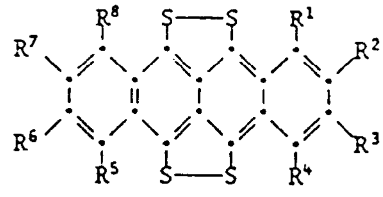
(III)

in which $R^1$ to $R^8$ independently of one another are H or a substituent belonging to the group -F, -Si($C_1$-$C_4$alkyl)$_3$ or -COOR$^{10}$; or each pair adjacent radicals of $R^1$ to $R^8$ is -CO-O-CO-; or $R^1$ to $R^8$ are a substituent belonging to the group $C_1$-$C_{20}$alkyl-$(X)_p$-, $C_3$-$C_8$cycloalkyl-$(X)_p$-, $C_1$-$C_{12}$alkyl-$C_3$-$C_8$cycloalkyl-$(X)_p$-, $C_3$-$C_8$cycloalkyl-$CH_2$-$(X)_p$-, $C_1$-$C_{12}$-alkyl-$C_3$-$C_8$cycloalkyl-$CH_2$-$(X)_p$-, $C_6$-$C_{10}$aryl-X-, $C_7$-$C_{20}$alkaryl-X-, $C_7$-$C_{12}$aralkyl-$(X)_p$- or $C_8$-$C_{20}$alkaralkyl-$(X)_p$- or -Y-$(C_mH_{2m}$-O$)_n$-$R^{10}$ each of which is unsubstituted or substituted by -F, -OH, $C_1$-$C_{12}$alkoxy, $C_1$-$C_{12}$acyloxy or - COOR$^{10}$; $R^{10}$ is H or $C_1$-$C_{18}$alkyl, X is -O-, -S-, -SO- or -$SO_2$- and Y is -O- or -S-, and p is 0 or 1, m is a number from 2 to 6 and n is a number from 2 to 20; which comprises reacting a compound of the formula Ia

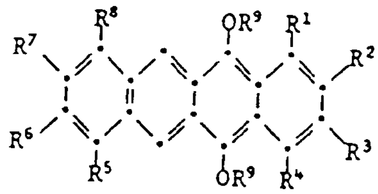

(Ia)

in which $R^1$ to $R^8$ are as defined above and $R^9$ is $C_1$-$C_4$acyl which is unsubstituted or substituted by -F, with sulfur in the presence of catalytic amounts of a sulfonic acid.

**13.** A process according to claim 12, wherein the reaction is carried out in the presence of a high-boiling solvent.

**14.** A process according to claim 12, wherein the reaction is carried out under an inert gas atmosphere.

**15.** A process according to claim 12, wherein the sulfonic acid is an aromatic sulfonic acid.

**16.** A process according to claim 15, wherein the sulfonic acid is p-toluenesulfonic acid.

**17.** A process according to claim 12, wherein the amount of the sulfonic acid is 0.001 to 10 mol %, relative to the amount of the compound of the formula Ia.

## Revendications

**1.** Composés de formule (I)

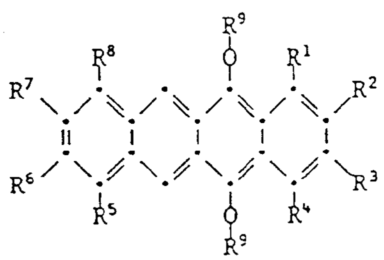

(I),

dans laquelle les symboles $R^1$ à $R^8$ représentent chacun, indépendamment l'un de l'autre, H et au moins l'un des symboles $R^1$ à $R^8$ représente un substituant choisi parmi -F, -Si (alkyle en $C_1$ à $C_4)_3$, $-COOR^{10}$; ou bien deux restes voisins choisis parmi $R^1$ à $R^8$ représentent -CO-O-CO-; ou bien l'un au moins des $R^1$ à $R^8$ représente un groupe alkyl (en $C_1$ à $C_{20}$)-$(X)_p$-, alcényl (en $C_2$ à $C_{20}$)-$(X)_p$-, alcynyl (en $C_2$ à $C_{20}$)-$(X)_p$, cycloalkyl(en $C_3$ à $C_8$)-$(X)_p$-, alkyl (en $C_1$ à $C_{12}$)-cycloalkyl(en $C_3$ à $C_8$)-$(X)_p$-, cycloalkyl(en $C_3$ à $C_8$)-$CH_2$-$(X)_p$-, alkyl(en $C_1$ à $C_{12}$)-cycloalkyl(en $C_3$ à $C_8$)-$CH_2$-$(X)_p$-, aryl(en $C_6$ à $C_{10}$)-(X)-, alcaryl(en $C_7$ à $C_{20}$)-X-, aralkyl(en $C_7$ à $C_{12}$)-$(X)_p$- ou alcaralkyl (en $C_8$ à $C_{20}$)-(X)-, (non substitués ou substitués par -F,-OH, -CN, par un groupe alcoxy en $C_3$ à $C_{12}$, acyloxy en $C_1$ à $C_{12}$ ou $-COR_{10}$) ou -Y-$(C_mH_{2m}$-O)n-$R^{10}$;

$R^{10}$ représente H ou un groupe alkyle en $C_1$ à $C_{18}$; X représente -O-, -S-, $-SO_2$ - et Y représente -O-ou -S-; p vaut 0 ou 1; m est un nombre valant 2 à 6 et n est un nombre valant 2 à 10; et $R^9$ représente un groupe acyle en $C_1$ à $C_4$, non substitué ou substitué par -F, à l'exclusion du 5,12-acétyloxy-7-méthyltétracène.

**2.** Composés selon la revendication 1, dans le cas desquels $R^1$ à $R^8$ représentent chacun , indépendamment l'un de l'autre, H et au moins l'un des symboles $R^1$ à $R^8$ représente un substituant choisi parmi -F, $-Si(CH_3)_3$, - COO(alkyle en $C_1$ à $C_{12}$), un groupe alkyle (en $C_1$ à $C_{18}$)- $(X)_p$, alcényl(en $C_{12}$ à $C_{18}$)-$(X)_p$-, alcynyl(en $C_2$ à $C_{12}$)-$(X)_p$-, aryl(en $C_6$ à $C_{10}$)-X-, alcaryl en $C_7$ à $C_{18}$)-X-, arakyl (en $C_7$ à $C_{10}$ )-$(X)_p$-, alcaralkyl(en $C_8$ à $C_{18}$)-$(X)_p$- ou $Y(C_mH_2$-$O)_n$-$R^{10}$ (non substitués ou substitués par -F, un groupe alcoxy en $C_1$ à $C_6$) alcyloxy en $C_1$ à $C_6$ ou -COO(alkyle en $C_1$ à $C_{12}$)); $R^{10}$ représente H ou un groupe alkyle en $C_1$ à $C_6$; X représente -O-, -S-, -SO- ou $-SO_2$- et Y représente -O- ou -S-; m vaut 2 ou 3, et n représente un nombre valant 2 à 12; et p vaut 0 ou 1; et $R^9$ représente un groupe acétyle non substitué ou substitué par -F.

3. Composés selon la revendication 1, dans lesquels $R^4$, $R^5$ et $R^8$ représentent chacun H.

4. Composés selon la revendication 1, dans lesquels $R^1$, $R^4$, $R^5$ et $R^8$ représentent chacun H.

5. Composés selon la revendication 1 dans lesquels $R^2$ et/ou $R^3$ représente(nt) un substituant et $R^1$ et $R^4$ à $R^8$ représentent chacun H.

6. Composés selon la revendication 1, dans lesquels $R^6$ et/ou $R^7$ représente(nt) un substituant et $R^1$ à $R^5$ et $R^8$ représentent H.

7. Composés selon la revendication 1 dans lesquels $R^1$ représente comme substituant $-CH_3$ ou un groupe -COO(alkyle en $C_1$ à $C_{12}$).

8. Composés selon la revendication 1, dans lesquels $R^1$ et $R^4$ à $R^8$ représentent chacun H; $R^2$ représente -F, $-CF_3$, un groupe -COO(alkyle en $C_1$ à $C_{18}$), un groupe alkyl (en $C_1$ à $C_{18}$)-X (non substitué ou substitué par -OH, par un groupe -COO(alkyl en $C_1$ à $C_6$) ou acyloxy en $C_1$ à $C_6$); $-Y-(CH_2CH_2O)n-R^{10}$, formule dans laquelle Y représente -O- ou -S-; $R^{10}$ représente H ou un groupe alkyl en $C_1$ à $C_6$ et n est un nombre valant 2 à 12; un groupe alcényl (en $C_2$ à $C_{12}$)-$CH_2$-X-; alcynyl (en $C_2$ à $C1_2$)-$CH_2$-X-; phényl -X; alkyl(en $C_1$ à $C_{12}$)phényl-X-; benzyl-X-; alkyl(en $C_1$ à $C_{12}$)-benzyl-X; et $R^3$ représente H ou a indépendamment, le sens de $R^2$; X représente -O-, -S-, -SO- ou $-SO_2-$ .

9. Procédé pour préparer des composés de formule I selon la revendication 1, caractérisé en ce qu'on fait réagir dans un solvant une naphtacène-5,12-dione substituée, de formule (II)

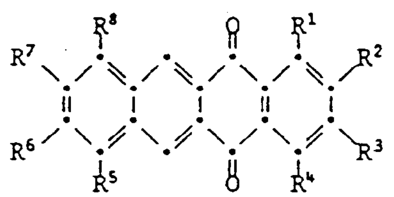
(II),

(dans laquelle $R^1$ à $R^8$ ont les sens indiqués ci-dessus) dans des conditions réductrices avec un anydride de formule $R^9CO-O-OCR^9$, dans laquelle $R^9$ a le sens indiqué à la revendication 1.

10. Procédé selon la revendication 9, caractérisé en ce que le solvant est un ester d'acide carboxylique.

11. Procédé selon la revendication 9, caractérisé en ce que les conditions réductrices sont obtenus par voie électro-chimique, catalytique ou par addition de métaux réducteurs.

12. Procédé pour préparer des 5,6-11,12-tétrathiotétracènes de formule III

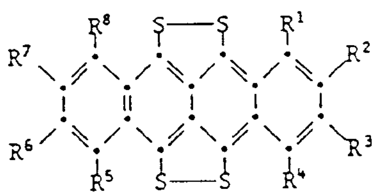
(III),

[dans laquelle $R^1$ à $R^8$ représente chacun, indépendamment l'un de l'autre H ou un substituant choisi parmi -F, un groupe -F, -Si(alkyl en $C_1$ à $C_4$)3, $-COOR^{10}$ ou bien deux restes voisins pris parmi $R^1$ à $R^8$ forment -CO-O-CO-; ou bien $R^1$ à $R^8$ représente chacun un substituant choisi parmi un groupe alkyl (en $C_1$ à $C_{20}$)-$(X)_p$-, cycloalkyl(en $C_1$ à $C_8$-)$(X)_p$-, alkyl(en $C_1$ à $C_{12}$)-cyclo(en $C_3$ à $C_8$)-$(X)_p$-, cycloalkyl(en $C_3$ à $C_8$)-$CH_2$-$(X)_p$-, alkyl(en $C_1$ à $C_{12}$)-cycloalkyl(en $C_3$ à $C_8$)-$CH_2$-$(X)_p$-, aryl(en $C_6$ à $C_{10}$)-X-, alcaryl(en $C_7$ à $C_{10}$)-X-, aralkyl(en $C_7$ à $C_{12}$)-$(X)_p$- ou alcaralkyl(en $C_1$ à $C_{20}$)-(X)-$_p$ [non substitués ou substitués par -F, -OH, ou un groupe alcoxy en $C_1$ à $C_{12}$, alcyoxy en $C_1$ à $C_{12}$ ou $-COOR^{10}$) ou -Y -S et $C_mH_{2m}$-O-$)_n$-$R^{10}$;

$R^{10}$ représente H ou un groupe alkyle en $C_1$ à $C_{18}$; X représente -O-, ou -S-, -SO-, ou $-SO_2-$ et Y

représente -O-S-; p vaut 0 ou 1; m est un nombre valant 2 à 6 et n est un nombre valant 2 à 20], procédé caractérisé en ce qu'on fait réagir avec du soufre un composé de formule I(a)

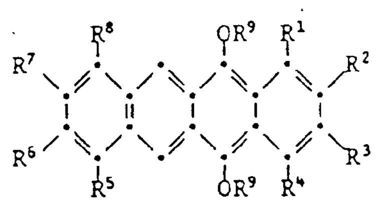

(Ia),

[dans laquelle $R^1$ à $R^8$ ont les sens indiqués ci-dessus et $R^9$ représente un groupe acyle en $C_1$ à $C_4$, non substitué et substitué par -F), en présence de quantités catalytiques d'un acide sulfonique.

**13.** Procédé selon la revendication 12, caractérisé en ce qu'on conduit la réaction en présence d'un solvant à point élevé d'ébullition.

**14.** Procédé selon la revendication 12, caractérisé en ce qu'on conduit la réaction en atmosphère de gaz inerte.

**15.** Procédé selon la revendication 12, caractérisé en ce qu'il s'agit, pour l'acide sulfonique, d'un acide sulfonique aromatique.

**16.** Procédé selon la revendication 15, caractérisé en ce qu'il s'agit de l'acide p-toluène sulfonique.

**17.** Procédé selon la revendication 12, caractérisé en ce que la quantité de l'acide sulfonique représente 0,01 à 10 moles % , par rapport à la quantité du composé de formule (Ia).